# EUROPEAN PATENT APPLICATION

(11) **EP 2 873 412 A1**
(43) Date of publication of application: **20.05.2015**
(21) Application number: 13192660.2
(22) Date of filing: 13.11.2013
(51) Int. Cl.: A61K 8/41, A61K 8/49, A61Q 5/10, A61K 8/66, A61K 8/22, A61K 8/26, A61K 8/27, A61K 8/19

(54) **A hair colour composition and method of colouring hair**

(71) Applicant: Unilever PLC, London, Greater London EC4Y 0DY (GB)
(72) Inventor: Anastasiadi, Maria, Sharnbrook, Bedford Mk44 1NX (GB)
(74) Representative: Warner, Guy Jonathan

(57) **Abstract**

This invention relates to a hair colour composition and method of colouring hair, in particular to a hair colour composition comprising a catecholamine for modifying the colour and uptake by keratinous fibres of a hair colour composition based on oxidation of a flavonoid.

The oxidation of flavonoids by peroxidase enzymes is known to colour hair fibres. This invention uses that system and introduces a new class of compounds (catecholamines) to produce a darker colour. Thus in a first aspect of the invention, a hair colour composition is provided, the hair colour composition comprising:
(a) A flavonoid of structure (I): 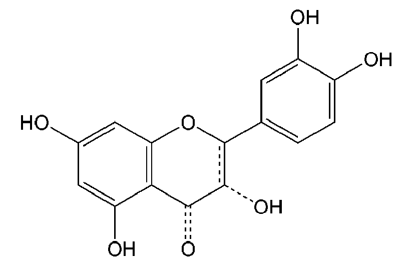 (b) A catecholamine of structure (II): 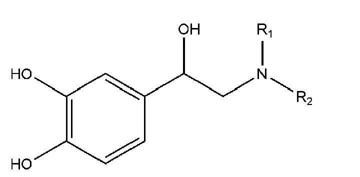 wherein R₁ and R₂ are independently H, C₁₋₅, C₁₋₃, C₁₋₂ branched or unbranched, substituted or unsubstituted alkyl moity, wherein the substituent is a halide, -OH, - NH₂ or =O;
(c) A peroxidase enzyme; and
(d) A hydrogen peroxide generator or hydrogen peroxide.

Further aspects of the invention are also described.

## Description

This invention relates to a hair colour composition and method of colouring hair, in particular to a hair colour composition comprising a catecholamine for modifying the colour and uptake by keratinous fibres of a hair colour composition based on oxidation of a flavonoid.

The oxidation of flavonoids by peroxidase enzymes is known to colour hair fibres. This invention uses that system and introduces a new class of compounds (catecholamines) to produce a darker colour.

US 6 953 486 B2 (L'Oreal) discloses a colouring composition (for colouring skin and/or keratin) comprising, in a physiologically acceptable medium, an efficient amount of at least one colouring agent precursor selected among the compounds comprising at least one aromatic cycle having at least two hydroxyl groups carried by two consecutive carbon atoms of the aromatic cycle, optionally an amino acid comprising at least one thiol group and an efficient amount of a catalytic system comprising a first component selected amongst salts and oxides of Mn(II) and/or Zn(II) and/or the mixtures thereof and a second component selected amongst alkaline hydrogenocarbonates, alkaline earth hydrogenocarbonates and the mixtures thereof, the proportions of the first component and the second component being such that: [Mn(II)] / [HCO₃] less than or equal to 1 with [Mn(II)] not equal to 0, [Zn(II)] / [HCO₃] less than or equal to 1 with [Zn(II)] not equal to 0, and [Mn(II) + Zn(II)] / [HCO₃] less than or equal to 1 with [Mn(II)] and [Zn(II)] not equal to 0.

Therefore it is possible to obtain colouring compositions comprising at least one colouring agent precursor adapted to self-colour, in the presence of oxygen, through oxidation by means of an enzymatic system (oxidase) and, optionally, at least one amino acid comprising at least one thiol group (SH), by replacing the enzymatic system by a purely chemical system. Thus, the chemical catalytic system behaves as a pseudo-oxidase able to simulate the oxidase activity without the inconvenients associated with the use of an enzymatic system. A preferred colouring agent precursor includes flavonols such as catechin. A preferred amino acid includes cysteine. The combination of L-DOPA, as a colouring agent precursor and cysteine, as an amino acid, is disclosed.

US 5 518 506 (L'Oreal) discloses that the use of certain para-aminophenols substituted in position 2 as oxidation dye precursors in combination with 6-hydroxyindole or 7-hydroxyindole and of an oxidising agent make it possible to obtain, in alkaline medium, after application to keratinous fibres and in particular human hair, a wide range of colourings with warm shades exhibiting a resistance to light, to washings and to bad weather, to perspiration and to the various treatments which hair may be subjected to. Example of the certain para-aminophenols includes 2-methoxymethyl-4-aminophenol and 2-ethoxymethyl-4-aminophenol.

US 5 135 544 (L'Oreal) discloses that by combining one or more particular monohydroxyindole(s) with one or more 5,6-disubstituted hydroxyindole(s), this application being followed by a rinse and then by the application of a composition containing, by way of an oxidizing agent, periodic acid or one or its water-soluble salts, miscellaneous colorations are obtained, especially on hair sensitized by permanent waving, these colorations showing especially little selectivity, withstanding successive washes, exhibiting good binding and not staining the scalp. Among the preferred monohydroxyindoles are 4-hydroxyindole, 5-hydroxyindole, 6-hydroxyindole and 7-hydroxyindole. 5,6-Dihydroxyindole is an especially preferred 5,6-disubstituted hydroxyindole.

US 7 776 105 B2 (Kao Corporation) discloses a hair dye composition containing a melanin precursor prepared efficiently by using an enzyme, which has excellent dyeing properties, stability and safety, in particular, a one-part air-oxidative hair dye composition. In particular, there is provided an air-oxidative type hair dye composition containing a melanin precursor prepared by a process including an oxidation step for converting, into the melanin precursor, a tyrosine or derivative thereof, which is used as a starting substance, with an enzyme or cell derived from a fungus selected from the group consisting of fungi belonging to the genera Aspergillus, Neurospora, Rhizomucor, Trichoderma, and Penicillium and exhibiting a catechol oxidase activity.

WO 2012/175720 A1 (L'Oreal) discloses The present invention relates to a process for dyeing keratin fibres, in particular human keratin fibres such as the hair, in which said fibres are treated with one or more cosmetic compositions containing, taken together or separately in said composition(s), the following ingredients: a) one or more dihydroxyflavonoid, neoflavanol or neoflavanone derivative(s); b) one or more manganese salt(s); c) hydrogen peroxide or one or more hydrogen peroxide-generating system(s); d) one or more (bi)carbonate(s) or one or more bicarbonate-generating system(s); e) one or more alkalinizing agent(s) different from the bicarbonate(s); and f) one or more metal salt(s) chosen from molybdenum, aluminium, zinc, calcium and rhodium salts; it being understood that the pH of at least one of the compositions comprising at least one of the ingredients a), b), d), e) and/or f) is alkaline and the metal salt(s) f) is (are) applied in the final step of the dyeing process. Dihydroxyflavonoids such as catechin, luteolin, quercetin amongst many others are mentioned. The dyeing process may use one or more ortho-diphenol derivative(s) different from the dihydroxyflavonoid, neoflavanol or neoflavanone derivative(s).

WO 2012/175684 A1 (L'Oreal) discloses a process for dyeing keratin fibres, in particular human keratin fibres such as the hair, in which said fibres are treated with one or more cosmetic compositions containing, taken together or separately in said composition(s), the following ingredients: a) one or more dihydroxyflavanol derivative(s), b) one or more manganese salt(s) or one or more zinc salt(s), c) hydrogen peroxide or one or more hydrogen peroxide- generating system(s), d) one or more (bi)carbonate(s) or one or more (bicarbonate- generating system(s), e) one or more alkalinizing agent(s) other than the bicarbonate(s), and f) one or more metal salt(s) chosen from magnesium salts, molybdenum salts and calcium salts; it being understood that the pH of at least one of the compositions comprising at least one of the ingredients a), b), d), e) and/or f) is alkaline and that the ingredient f) is applied together with at least one of the other ingredients a) to e). Preferably the dihydroxyflavanol derivative is catechin. The dyeing process may use one or more ortho-diphenol derivative(s) different from the dihydroxyflavonol derivative(s) a).

EP 0 341 808 A1 (Bristol-Myers Company) discloses a process for colouring hair in which a functional metal ion and aminoalkyl or aminohydroxyalkyl catechols are used in combination or applied sequentially to dye the hair. Specific examples of suitable aminoalkyl or aminohydroxyalkyl catechols are 3,4-dihydroxyphenylethylamine (Dopamine), epinephrine, 3,4-dihydroxybenzylamine and 2-hydroxy-2-(3,4-dihydroxyphenyl)ethylamine.

EP 0 161 073 A2 (Repligen Corporation) discloses a dye composition comprises (1) a suitable organic compound to assist dye penetration, (2) a dye precursor which is dopamine, D-DOPA, L- DOPA, D,L- DOPA or an analogue thereof, and (3) iodate or periodate. The composition can be used to colour hair or other keratinous fibres, e.g. from gray to black. The most preferred dye precursor is dopamine. Examples of suitable analogues of dopamine, D- DOPA, L- DOPA, or D,L-DOPA are 2-methyldopamine, 5-methyldopamine, or alpha-(aminomethyl)-3,4-dihydroxybenzyl alcohol.

US 8 366 791 B1 (Warner Babcock Institute) discloses a colouring formulation including a catechol-based precursor and an oxidizing agent. Additionally an alkalizing agent may be present. The formulation is substantially free of organic solvents, co-solvents and diluents. The catechol-based dye precursor may be chosen from L-DOPA, D-DOPA or pharmaceutically acceptable salts thereof and/or esters thereof or mixtures thereof The catechol-based dye precursor may also be of formula: 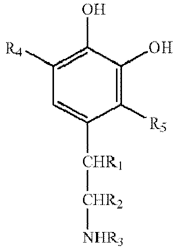 wherein R₁ and R₂ can be the same or different and are: H, alkyl or 1-4C, NH₂, OH, COOR' wherein R' is alkyl of 1-4C or H, CONH₂, halogen, OR" wherein R" is alkyl of 1-4C, CH₂OH, CH₂NH₂, CONR'R" wherein R' and R" can be the same or different; R₃ is H or alkyl of 1-4C or COR"; R₄, R₅ can be the same or different and are: H, alkyl of 1-4C, NH₂, OH, COOH, CONH₂, halogen, OR", NO₂, SO₃, HNR" or NR"R".

### Summary of the Invention

In a first aspect of the invention, a hair colour composition is provided, the hair colour composition comprising:
(a) A flavonoid of structure (I): 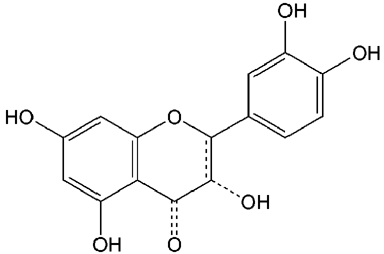
(b) A catecholamine of structure (II): 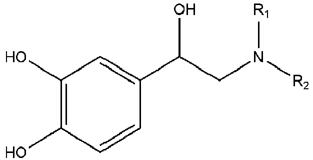 wherein R₁ and R₂ are independently H, C₁₋₅, C₁₋₃, C₁₋₂ branched or unbranched, substituted or unsubstituted alkyl moity, wherein the substituent is a halide, -OH,-NH₂ or =O;
(c) A peroxidase enzyme; and
(d) A hydrogen peroxide generator or hydrogen peroxide.

In a second aspect of the invention, a kit for colouring hair is provided, the kit for colouring hair comprising:
(a) A hair colour composition according to the first aspect of the invention; and
(b) A pre- or post-treatment composition comprising a metal ion suitable for coordinating to the flavonoid of structure (I) or the product of the reaction of technical features (a), (b), (c) and (d) of the first aspect of the invention.

In a third aspect of the invention, a method for colouring hair is provided, the method for colouring hair comprising the step of treating hair with the hair composition of the first aspect of the invention.

### Detailed Description of the Invention

In a first aspect of the invention, a hair colour composition is provided, the hair colour composition comprising:
(a) A flavonoid of structure (I): 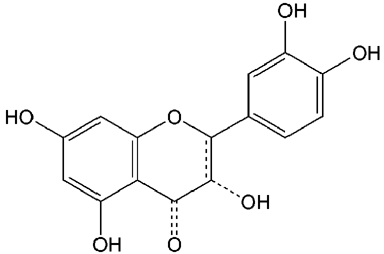
(b) A catecholamine of structure (II): 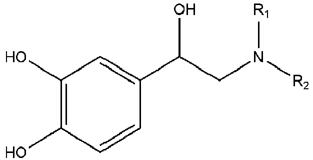 wherein R₁ and R₂ are independently H, C₁₋₅, C₁₋₃, C₁₋₂ branched or unbranched, substituted or unsubstituted alkyl moity, wherein the substituent is a halide, -OH,-NH₂ or =O;
(c) A peroxidase enzyme; and
(d) A hydrogen peroxide generator or hydrogen peroxide.

Preferably the flavonoid is selected from the group consisting of catechin, luteolin, quercetin, taxifolin and epicatechin. More preferably the flavonoid is (+)-catechin or (-)-catechin. More preferably the flavonoid may be (+)-epicatechin or (-)-epicatechin. The hair colour composition may comprise 0.01 to 10, preferably 0.1 to 5 % w/w flavonoid of structure (I).

Preferably R₁ on the catecholamine of structure (II) is -H. Preferably R₂ on the catecholamine of structure (II) is a C₁₋₃ substituted or unsubstituted alkyl group or -H, more preferably a methyl group or -H. The catecholamine may be selected from the group consisting of epinephrine, isoprenaline and norepinephrine. The hair colour composition may comprise 0.01 to 10, preferably 0.1 to 5 % w/w compound of structure (II).

A hair colour composition according to any one of the preceding claims wherein the molar ratio of flavonoid of structure (I) to compound of structure (II) is 1:1 to 120, preferably 1:2-1:15, most preferably 1:3 to 1:10. It has been observed that the colour taken up on hair is not as dark if the compound of structure (I) is in excess, or if the compound of structure (II) is in large excess (greater than 10-fold on a molar basis). The darkest colours formed were observed when the molar ratio of flavonoid of structure (I) to compound of structure (II) was 1:3 to 1:10.

The peroxidase may be a non-animal haem peroxidase from class II (fungi) or class III (plants and algae). In particular the peroxidase may be obtained from the group consisting of Arabidopsis thaliana, horse radish, barley, peanut soy bean, tobacco, and turnip (plants), Chlorophyta spirogyra (green algae), Arthromyces ramosus and Corprinus cinereus (fungi). Preferably the peroxidase is horse radish peroxidase or soy bean peroxidase. The hair colour composition may comprise 0.0001 to 5, preferably 0.001 to 1 % w/w peroxidase.

The hair colour composition may comprise 0.0001 to 3, preferably 0.001 to 1, most preferably 0.01 to 1 % w/w hydrogen peroxide.

The hydrogen peroxide generator may comprise a hydrogen peroxide generating oxidase, a substrate and oxygen. The hydrogen peroxide generating oxidase may be selected from the group consisting of (S)-2-hydroxy acid oxidase, D-galactose oxidase, glucose oxidase, coniferyl alcohol oxidase, glycolate oxidase, hexose oxidase, oxalate oxidase, amino acid oxidase and L-galactonolactone oxidase and the respective substrate is selected from the group consisting of (S)-2-hydroxy acid, D-galactose, glucose, coniferyl alcohol, α-hydroxy acids, D-glucose, oxalic acid, amino acid and L-galactono-1,4-lactone.

Thus the hydrogen peroxide generator may be selected from the group consisting of (S)-2-hydroxy acid with (S)-2-hydroxy acid oxidase, D-galactose with D-galactose oxidase, glucose with glucose oxidase, coniferyl alcohol with coniferyl alcohol oxidase, α-hydroxy acids with glycolate oxidase, D-glucose with hexose oxidase, oxalic acid with oxalate oxidase, and L-galactono-1,4-lactone with L-galactonolactone oxidase, amino acid oxidase with amino acids, all in the presence of oxygen.

The hair colour composition may have a pH of 5-9, preferably 5-8, most preferably 5-7.

The hair colour composition may take the form of a shampoo or hair conditioning composition, or a 2-in-1 conditioning shampoo composition.

### Shampoo Compositions

Shampoo compositions will nearly always comprise a cleansing surfactant component in an aqueous base.

### Cleansing Surfactant

The cleansing surfactant may consist of a single surfactant, usually an anionic surfactant (to provide foam) such as sodium lauryl ether sulphate, or more commonly a mixture of sodium lauryl ether sulphate with a co-surfactant to provide mildness. The most preferred co-surfactant is cocoamidopropyl betaine.

The total amount of surfactant (including any co-surfactant, and/or any emulsifier) in a shampoo composition may be from 1 to 50, preferably from 2 to 40, more preferably from 10 to 25 % w/w. Compositions comprising more than 25 % w/w cleansing surfactant are commonly considered concentrated shampoos.

Examples of suitable anionic cleansing surfactants are the alkyl sulphates, alkyl ether sulphates, alkaryl sulphonates, alkanoyl isethionates, alkyl succinates, alkyl sulphosuccinates, alkyl ether sulphosuccinates, N-alkyl sarcosinates, alkyl phosphates, alkyl ether phosphates, and alkyl ether carboxylic acids and salts thereof, especially their sodium, magnesium, ammonium and mono-, di- and triethanolamine salts. The alkyl and acyl groups generally contain from 8 to 18, preferably from 10 to 16 carbon atoms and may be unsaturated. The alkyl ether sulphates, alkyl ether sulphosuccinates, alkyl ether phosphates and alkyl ether carboxylic acids and salts thereof may contain from 1 to 20 ethylene oxide or propylene oxide units per molecule.

Typical anionic cleansing surfactants for use in shampoo compositions of the invention include sodium oleyl succinate, ammonium lauryl sulphosuccinate, sodium lauryl sulphate, sodium lauryl ether sulphate, sodium lauryl ether sulphosuccinate, ammonium lauryl sulphate, ammonium lauryl ether sulphate, sodium dodecylbenzene sulphonate, triethanolamine dodecylbenzene sulphonate, sodium cocoyl isethionate, sodium lauryl isethionate, lauryl ether carboxylic acid and sodium N-lauryl sarcosinate.

Preferred anionic surfactants are the alkyl sulfates and alkyl ether sulfates. These materials have the respective formulae R2OSO₃M and R1O(C₂H₄O)xSO₃M, wherein R2 is alkyl or alkenyl of from 8 to 18 carbon atoms, x is an integer having a value of from about 1 to about 10, and M is a cation such as ammonium, alkanolamines, such as triethanolamine, monovalent metals, such as sodium and potassium, and polyvalent metal cations, such as magnesium, and calcium. Most preferably R2 has 12 to 14 carbon atoms, in a linear rather than branched chain.

Preferred anionic cleansing surfactants are selected from sodium lauryl sulphate and sodium lauryl ether sulphate(n)EO, (where n is from 1 to 3); more preferably sodium lauryl ether sulphate(n)EO, (where n is from 1 to 3); most preferably sodium lauryl ether sulphate(n)EO where n=1.

Preferably the level of alkyl ether sulphate is from 0.5 to 25, more preferably from 3 to 18, most preferably from 6 to 15 % w/w of the composition.

The total amount of anionic cleansing surfactant in shampoo compositions of the invention generally ranges from 0.5 to 45, more preferably from 1.5 to 20 % w/w of the composition.

### Nonionic Surfactant

Shampoo compositions of the invention may contain non-ionic surfactant. Most preferably non-ionic surfactants are present in the range 0 to 5 % w/w of the composition.

Nonionic surfactants that can be included in shampoo compositions of the invention include condensation products of aliphatic (C8 - C18) primary or secondary linear or branched chain alcohols or phenols with alkylene oxides, usually ethylene oxide and generally having from 6 to 30 ethylene oxide groups. Alkyl ethoxylates are particularly preferred. Most preferred are alkyl ethoxylates having the formula R-(OCH2CH2)nOH, where R is an alkyl chain of C12 to C15, and n is 5 to 9.

Other suitable nonionic surfactants include mono- or di-alkyl alkanolamides. Examples include coco mono- or di-ethanolamide and coco mono-isopropanolamide.

Further nonionic surfactants which can be included in shampoo compositions of the invention are the alkyl polyglycosides (APGs). Typically, APG is one which comprises an alkyl group connected (optionally via a bridging group) to a block of one or more glycosyl groups. Preferred APGs are defined by the following formula RO - (G)n wherein R is a branched or straight chain alkyl group which may be saturated or unsaturated and G is a saccharide group.

R may represent a mean alkyl chain length of from about C5 to about C20. Preferably R represents a mean alkyl chain length of from about C8 to about C12. Most preferably the value of R lies between about 9.5 and about 10.5. G may be selected from C5 or C6 monosaccharide residues, and is preferably a glucoside. G may be selected from the group comprising glucose, xylose, lactose, fructose, mannose and derivatives thereof. Preferably G is glucose. The degree of polymerisation, n, may have a value of from about 1 to about 10 or more, preferably a value of from about 1.1 to about 2, most preferably a value of from about 1.3 to about 1.5. Suitable alkyl polyglycosides for use in the invention are commercially available and include for example those materials identified as: Oramix NS10 ex Seppic; Plantaren 1200 and Plantaren 2000 ex Henkel.

Other sugar-derived nonionic surfactants which can be included in compositions of the invention include the C10-C18 N-alkyl (C1-C6) polyhydroxy fatty acid amides, such as the C12-C18 N-methyl glucamides, as described for example in WO 92/06154 and US 5 194 639, and the N-alkoxy polyhydroxy fatty acid amides, such as C10-C18 N-(3-methoxypropyl) glucamide.

### Amphoteric/zwitterionic Surfactant

Amphoteric or zwitterionic surfactant can be included in an amount ranging from 0.5 to about 8, preferably from 1 to 4 % w/w of the shampoo compositions of the invention.

Examples of amphoteric or zwitterionic surfactants include alkyl amine oxides, alkyl betaines, alkyl amidopropyl betaines, alkyl sulphobetaines (sultaines), alkyl glycinates, alkyl carboxyglycinates, alkyl amphoacetates, alkyl amphopropionates, alkylamphoglycinates, alkyl amidopropyl hydroxysultaines, acyl taurates and acyl glutamates, wherein the alkyl and acyl groups have from 8 to 19 carbon atoms. Typical amphoteric and zwitterionic surfactants for use in shampoos of the invention include lauryl amine oxide, cocodimethyl sulphopropyl betaine, lauryl betaine, cocamidopropyl betaine and sodium cocoamphoacetate.

A particularly preferred amphoteric or zwitterionic surfactant is cocamidopropyl betaine.

Mixtures of any of the foregoing amphoteric or zwitterionic surfactants may also be suitable. Preferred mixtures are those of cocamidopropyl betaine with further amphoteric or zwitterionic surfactants as described above. A preferred further amphoteric or zwitterionic surfactant is sodium cocoamphoacetate.

### Suspending agents

Preferably an aqueous shampoo composition of the invention further comprises a suspending agent. Suitable suspending agents are selected from polyacrylic acids, cross-linked polymers of acrylic acid, copolymers of acrylic acid with a hydrophobic monomer, copolymers of carboxylic acid-containing monomers and acrylic esters, cross-linked copolymers of acrylic acid and acrylate esters, heteropolysaccharide gums and crystalline long chain acyl derivatives. The long chain acyl derivative is desirably selected from ethylene glycol stearate, alkanolamides of fatty acids having from 16 to 22 carbon atoms and mixtures thereof. Ethylene glycol distearate and polyethylene glycol 3 distearate are preferred long chain acyl derivatives, since these impart pearlescence to the composition. Polyacrylic acid is available commercially as Carbopol 420, Carbopol 488 or Carbopol 493. Polymers of acrylic acid cross-linked with a polyfunctional agent may also be used; they are available commercially as Carbopol 910, Carbopol 934, Carbopol 941 and Carbopol 980. An example of a suitable copolymer of a carboxylic acid containing monomer and acrylic acid esters is Carbopol 1342. Carbopol 980 is the commonly used suspending agent though there is a growing desire to find an alternative. All Carbopol (trademark) materials are available from Goodrich.

Suitable cross-linked polymers of acrylic acid and acrylate esters are Pemulen TR1 or Pemulen TR2. A suitable heteropolysaccharide gum is xanthan gum, for example that available as Kelzan mu.

Mixtures of any of the above suspending agents may be used. Preferred is a mixture of cross-linked polymer of acrylic acid and crystalline long chain acyl derivative.

Suspending agent will generally be present in a shampoo composition of the invention at levels of from 0.1 to 10, preferably from 0.5 to 6, more preferably from 0.9 to 4 % w/w of the composition. Generally such suspending agents are present at around 2 % w/w of the composition.

### Water

Shampoo compositions of the invention are generally aqueous, i.e. they have water or an aqueous solution or a lyotropic liquid crystalline phase as their major component. Suitably, the composition will comprise from 50 to 98, preferably from 60 to 90 % w/w of the composition.

Typically, shampoo compositions have a pH of around 5.5.

### Optional Ingredients

The shampoo compositions of the invention might also contain the following optional ingredients: conditioning agents.

### (a) Conditioning Agents

Conditioning actives are often included in shampoo compositions. These are sometimes called '2-in-1' formulations. Conditioning actives fall into three classes:
- silicones (and cationic deposition polymers to assist in silicone deposition)
- cationic surfactants
- non-silicone oils

Where silicones are included, the composition is likely to also contain a cationic deposition polymer for enhancing deposition of the silicone. Further, a silicone-containing composition is likely to be lamellar as opposed to isotropic. Isotropic compositions do not deposit silicone effectively.

### Silicones

The shampoo compositions of the invention can contain emulsified droplets of a silicone conditioning agent, for enhancing conditioning performance.

Suitable silicones include polydiorganosiloxanes, in particular polydimethylsiloxanes which have the CTFA designation dimethicone. Also suitable for use compositions of the invention (particularly shampoos and conditioners) are polydimethyl siloxanes having hydroxyl end groups, which have the CTFA designation dimethiconol. Also suitable for use in compositions of the invention are silicone gums having a slight degree of cross-linking, as are described for example in WO 96/31188.

Examples of suitable pre-formed emulsions include Xiameter MEM 1785 and microemulsion DC2-1865 available from Dow Corning. These are emulsions /microemulsions of dimethiconol. Cross-linked silicone gums are also available in a pre-emulsified form, which is advantageous for ease of formulation.

A further preferred class of silicones for inclusion in shampoos and conditioners of the invention are amino functional silicones. By "amino functional silicone" is meant a silicone containing at least one primary, secondary or tertiary amine group, or a quaternary ammonium group. Examples of suitable amino functional silicones include: polysiloxanes having the CTFA designation "amodimethicone".

Specific examples of amino functional silicones suitable for use in the invention are the aminosilicone oils DC2-8220, DC2-8166 and DC2-8566 (all ex Dow Corning). The most commonly used amino silicone is sourced from Dow Corning and is coded DC7134. Pre-formed emulsions of amino functional silicone are also available from suppliers of silicone oils such as Dow Corning and General Electric. Specific examples include DC939 Cationic Emulsion and the non-ionic emulsions DC2-7224, DC2-8467, DC2-8177 and DC2-8154 (all ex Dow Corning).

Suitable quaternary silicone polymers are described in EP-A-0 530 974. A preferred quaternary silicone polymer is K3474, ex Goldschmidt.

With some shampoos it is preferred to use a combination of amino and non amino functional silicones.

Emulsified silicones for use in the shampoo compositions of the invention will typically have an average silicone droplet size in the composition of less than 30, preferably less than 20, more preferably less than 10 micron, ideally from 0.01 to 1 micron. Silicone emulsions having an average silicone droplet size of about 0.15 micron are generally termed microemulsions.

Emulsified silicones for use in the conditioner compositions of the invention will typically have a size in the composition of less than 30, preferably less than 20, more preferably less than 15. Preferably the average silicone droplet is greater than 0.5 micron, more preferably greater than 1 micron, ideally from 2 to 8 micron.

Silicone particle size may be measured by means of a laser light scattering technique, for example using a 2600D Particle Sizer from Malvern Instruments.

The viscosity of the emulsified silicone itself (not the emulsion or the final hair conditioning composition) is typically at least 10,000, preferably at least 60,000, most preferably at least 500,000, ideally at least 1,000,000 cst at 25 oC. Preferably the viscosity does not exceed 109 cst at 25 oC for ease of formulation.

The total amount of silicone is preferably from 0.01 to 10, more preferably from 0.1 to 5, most preferably 0.5 to 3 % w/w of the composition of the invention.

Also suitable are emulsions of amino functional silicone oils with non ionic and/or cationic surfactant.

Cationic deposition polymers are used to deposit the silicone droplets to the hair surface and hence enhance performance.

Suitable cationic polymers may be homopolymers which are cationically substituted or may be formed from two or more types of monomers. The weight average (Mw) molecular weight of the polymers will generally be between 100 000 and 2 million daltons. The polymers will have cationic nitrogen containing groups such as quaternary ammonium or protonated amino groups, or a mixture thereof. If the molecular weight of the polymer is too low, then the conditioning effect is poor. If too high, then there may be problems of high extensional viscosity leading to stringiness of the composition when it is poured.

The cationic nitrogen-containing group will generally be present as a substituent on a fraction of the total monomer units of the cationic polymer. Thus when the polymer is not a homopolymer it can contain spacer non-cationic monomer units. Such polymers are described in the CTFA Cosmetic Ingredient Directory. The ratio of the cationic to non-cationic monomer units is selected to give polymers having a cationic charge density in the required range, which is generally from 0.2 to 3.0 meq/gm. The cationic charge density of the polymer is suitably determined via the Kjeldahl method as described in the US Pharmacopoeia under chemical tests for nitrogen determination.

Suitable cationic polymers include, for example, copolymers of vinyl monomers having cationic amine or quaternary ammonium functionalities with water soluble spacer monomers such as (meth)acrylamide, alkyl and dialkyl (meth)acrylamides, alkyl (meth)acrylate, vinyl caprolactone and vinyl pyrrolidine. The alkyl and dialkyl substituted monomers preferably have C1-C7 alkyl groups, more preferably C1-3 alkyl groups. Other suitable spacers include vinyl esters, vinyl alcohol, maleic anhydride, propylene glycol and ethylene glycol.

The cationic amines can be primary, secondary or tertiary amines, depending upon the particular species and the pH of the composition. In general secondary and tertiary amines, especially tertiary, are preferred.

Amine substituted vinyl monomers and amines can be polymerized in the amine form and then converted to ammonium by quaternization.

The cationic polymers can comprise mixtures of monomer units derived from amine- and/or quaternary ammonium-substituted monomer and/or compatible spacer monomers.

Suitable cationic polymers include, for example:
- cationic diallyl quaternary ammonium-containing polymers including, for example, dimethyldiallylammonium chloride homopolymer and copolymers of acrylamide and dimethyldiallylammonium chloride, referred to in the industry (CTFA) as Polyquaternium 6 and Polyquaternium 7, respectively;
- mineral acid salts of amino-alkyl esters of homo-and co-polymers of unsaturated carboxylic acids having from 3 to 5 carbon atoms, (as described in US 4 009 256); and
- cationic polyacrylamides (as described in WO95/22311).

Other cationic polymers that can be used include cationic polysaccharide polymers, such as cationic cellulose derivatives, cationic starch derivatives, and cationic guar gum derivatives.

Cationic polysaccharide polymers suitable for use in compositions of the invention include monomers of the formula A-O-[R-N+(R1)(R2)(R3)X-] wherein A is an anhydroglucose residual group, such as a starch or cellulose anhydroglucose residual; R is an alkylene, oxyalkylene, polyoxyalkylene, or hydroxyalkylene group, or combination thereof; R1, R2 and R3 independently represent alkyl, aryl, alkylaryl, arylalkyl, alkoxyalkyl, or alkoxyaryl groups, each group containing up to about 18 carbon atoms; the total number of carbon atoms for each cationic moiety (i.e., the sum of carbon atoms in R1, R2 and R3) is preferably about 20 or less; and X is an anionic counterion.

Another type of cationic cellulose includes the polymeric quaternary ammonium salts of hydroxyethyl cellulose reacted with lauryl dimethyl ammonium-substituted epoxide, referred to in the industry (CTFA) as Polyquaternium 24. These materials are available from the Amerchol Corporation, for instance under the tradename Polymer LM-200.

Other suitable cationic polysaccharide polymers include quaternary nitrogen-containing cellulose ethers (e.g. as described in US 3 962 418), and copolymers of etherified cellulose and starch (e.g. as described in US 3 958 581).

A particularly suitable type of cationic polysaccharide polymer that can be used is a cationic guar gum derivative, such as guar hydroxypropyltrimethylammonium chloride (commercially available from Rhodia in their JAGUAR trademark series). Examples of such materials are JAGUAR C13S, JAGUAR C14, JAGUAR C15, JAGUAR C17 and JAGUAR C16 Jaguar CHT and JAGUAR C162.

Mixtures of any of the above cationic polymers may be used.

Cationic polymer will generally be present in a shampoo composition of the invention at levels of from 0.01 to 5, preferably from 0.05 to 1, more preferably from 0.08 to 0.5 % w/w of the weight of the compositions of the invention.

### Cationic Surfactants

Cationic surfactants may be used in 2-in-1 shampoos to provide a conditioning benefit. However, since a shampoo composition is likely to also comprise anionic cleansing surfactants, the use of cationic surfactants is limited to compositions where the cationic surfactant is separated from the anionic phase by way of a stable conditioning gel phase made separately from the rest of the formulation and then incorporated afterwards.

### Non-silicone Oils

These are typically hydrocarbon oils or fatty alcohols. A fatty alcohol is nearly always included in a conditioning composition and often included in 2-in-1 shampoos. Cetearyl alcohol is one of the preferred examples.

### (b) Fibre Actives

Fibre actives are provided to repair or coat the hair fibres. Examples are trehalose (a disaccharide), adipic acid (dicarboxylic acid) and gluconolactone.

### (c) Anti-dandruff Actives

There are two classes of anti-dandruff active: the azoles and the pyrithiones, both are active against the target fungi malassezia spp. The azoles include ketoconazole and climbazole which are fat soluble actives. The pyrithiones include zinc pyrithione (ZPT) which is insoluble and delivered as a particle to the scalp.

Preferably, the antidandruff active is present at from 0.01 to 5, more preferably from 0.1 to 2.5 % w/w of the composition of the invention.

### Hair Conditioning Compositions

The compositions of the invention may also be hair conditioning compositions (also known as conditioners). A conditioner which is to be used after a shampoo is known as a 'system conditioner' whereas one which is included in a shampoo composition is known as a '2-in-1'. Hair conditioning compositions may also be left on the head, i.e. not rinsed off after application. These are known as Leave-on-Treatments (LOTs) as opposed to Rinse-off-Treatments (ROTs).

The main ingredients in a system conditioner are the conditioning actives described above, the main actives being a cationic surfactant (e.g. behenyltrimmonium chloride), a silicone conditioning agent (e.g. aminosilicone (DC 7134)) and a non-silicone oil, usually a fatty alcohol (e.g. cetearyl alcohol).

Anti-dandruff actives may also be included in hair conditioning compositions of the invention.

### Cationic Surfactants

Preferably, the cationic surfactants have the formula N+R1 R2R3R4 wherein R1, R2, R3 and R4 are independently (C1 to C30) alkyl or benzyl. Preferably, one, two or three of R1, R2, R3 and R4 are independently (C4 to C30) alkyl and the other R1, R2, R3 and R4 group or groups are (C1-C6) alkyl or benzyl. More preferably, one or two of R1, R2, R3 and R4 are independently (C6 to C30) alkyl and the other R1, R2, R3 and R4 groups are (C1-C6) alkyl or benzyl groups. Optionally, the alkyl groups may comprise one or more ester (-OCO- or -COO-) and/or ether (-O-) linkages within the alkyl chain. Alkyl groups may optionally be substituted with one or more hydroxyl groups. Alkyl groups may be straight chain or branched and, for alkyl groups having 3 or more carbon atoms, cyclic. The alkyl groups may be saturated or may contain one or more carbon-carbon double bonds (e.g. oleyl). Alkyl groups are optionally ethoxylated on the alkyl chain with one or more ethyleneoxy groups.

Suitable cationic surfactants for use in conditioner compositions according to the invention include cetyltrimethylammonium chloride, behenyltrimethylammonium chloride, cetylpyridinium chloride, tetramethylammonium chloride, tetraethylammonium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, didodecyldimethylammonium chloride, dioctadecyldimethylammonium chloride, tallowtrimethylammonium chloride, dihydrogenated tallow dimethyl ammonium chloride (eg, Arquad 2HT/75 from Akzo Nobel), cocotrimethylammonium chloride, PEG-2-oleammonium chloride and the corresponding hydroxides thereof. Further suitable cationic surfactants include those materials having the CTFA designations Quaternium-5, Quaternium-31 and Quaternium-18. Mixtures of any of the foregoing materials may also be suitable. A particularly useful cationic surfactant for use in conditioners according to the invention is cetyltrimethylammonium chloride, available commercially, for example as GENAMIN CTAC, ex Hoechst Celanese. Another particularly useful cationic surfactant for use in conditioners according to the invention is behenyltrimethylammonium chloride, available commercially, for example as GENAMIN KDMP, ex Clariant.

Another example of a class of suitable cationic surfactants for use in the invention, either alone or together with one or more other cationic surfactants, is a combination of (i) and (ii) below:
(i) an amidoamine corresponding to the general formula (I) R1CONH(CH₂)mN(R2)R3 in which R1 is a hydrocarbyl chain having 10 or more carbon atoms, R2 and R3 are independently selected from hydrocarbyl chains of from 1 to 10 carbon atoms, and m is an integer from 1 to about 10; and
(ii) an acid.

As used herein, the term hydrocarbyl chain means an alkyl or alkenyl chain. Preferred amidoamine compounds are those corresponding to formula (I) in which R1 is a hydrocarbyl residue having from about 11 to about 24 carbon atoms, R2 and R3 are each independently hydrocarbyl residues, preferably alkyl groups, having from 1 to about 4 carbon atoms, and m is an integer from 1 to about 4. Preferably R2 and R3 are methyl or ethyl groups. Preferably m is 2 or 3, i.e. an ethylene or propylene group.

Preferred amidoamines useful herein include stearamido-propyldimethylamine, stearamidopropyldiethylamine, stearamidoethyldiethylamine, stearamidoethyldimethylamine, palmitamidopropyldimethylamine, palmitamidopropyldiethylamine, palmitamidoethyldiethylamine, palmitamidoethyldimethylamine, behenamidopropyldimethyl-amine, behenamidopropyldiethylmine, behenamidoethyldiethylamine, behenamidoethyldimethylamine, arachidamidopropyl-dimethylamine, arachidamidopropyldiethylamine, arachid-amidoethyldiethylamine, arachidamidoethyldimethylamine, and mixtures thereof. Particularly preferred amidoamines useful herein are stearamidopropyldimethylamine, stearamidoethyldiethylamine, and mixtures thereof.

Commercially available amidoamines useful herein include: stearamidopropyldimethylamine with tradenames LEXAMINE S-13 available from Inolex (Philadelphia Pennsylvania, USA) and AMIDOAMINE MSP available from Nikko (Tokyo, Japan), stearamidoethyldiethylamine with a tradename AMIDOAMINE S available from Nikko, behenamidopropyldimethylamine with a tradename INCROMINE BB available from Croda (North Humberside, England), and various amidoamines with tradenames SCHERCODINE series available from Scher (Clifton New Jersey, USA).

The acid may be any organic or mineral acid which is capable of protonating the amidoamine in the conditioner composition. Suitable acids useful herein include hydrochloric acid, acetic acid, tartaric acid, fumaric acid, lactic acid, malic acid, succinic acid, and mixtures thereof. Preferably, the acid is selected from the group consisting of acetic acid, tartaric acid, hydrochloric acid, fumaric acid, lactic acid and mixtures thereof.

The primary role of the acid is to protonate the amidoamine in the hair treatment composition thus forming a tertiary amine salt (TAS) in-situ in the hair treatment composition. The TAS in effect is a non-permanent quaternary ammonium or pseudo-quaternary ammonium cationic surfactant. Suitably, the acid is included in a sufficient amount to protonate more than 95 mole % (20°C) of the amidoamine present.

In conditioners of the invention, the level of cationic surfactant will generally range from 0.01 to 10, more preferably 0.05 to 7.5, most preferably 0.1 to 5 % by weight of the composition.

### Silicone Conditioning Agent

The compositions of the invention can contain emulsified droplets of a silicone conditioning agent, for enhancing conditioning performance as previously described.

### Non-silicone Oils

Compositions according to the present invention may also comprise a dispersed, nonvolatile, water-insoluble, non-silicone oily conditioning agent. Preferably such non-silicone oily conditioning agents are present in the hair conditioning compositions of the invention. By "insoluble" is meant that the material is not soluble in water (distilled or equivalent) at a concentration of 0.1 % w/w at 25°C. Suitable non-silicone oils are selected from hydrocarbon oils, fatty esters and mixtures thereof.

Straight chain hydrocarbon oils will preferably contain from about 12 to about 30 carbon atoms. Also suitable are polymeric hydrocarbons of alkenyl monomers, such as C2-C6 alkenyl monomers. Specific examples of suitable hydrocarbon oils include paraffin oil, mineral oil, saturated and unsaturated dodecane, saturated and unsaturated tridecane, saturated and unsaturated tetradecane, saturated and unsaturated pentadecane, saturated and unsaturated hexadecane, and mixtures thereof. Branched-chain isomers of these compounds, as well as of higher chain length hydrocarbons, can also be used.

Suitable fatty esters are characterised by having at least 10 carbon atoms, and include esters with hydrocarbyl chains derived from fatty acids or alcohols, Monocarboxylic acid esters include esters of alcohols and/or acids of the formula R'COOR in which R' and R independently denote alkyl or alkenyl radicals and the sum of carbon atoms in R' and R is at least 10, preferably at least 20. Di- and trialkyl and alkenyl esters of carboxylic acids can also be used. Particularly preferred fatty esters are mono-, di- and triglycerides, more specifically the mono-, di-, and tri-esters of glycerol and long chain carboxylic acids such as C1-C22 carboxylic acids. Preferred materials include cocoa butter, palm stearin, sunflower oil, soyabean oil and coconut oil.

The oily or fatty material is suitably present at a level of from 0.05 to 10, preferably from 0.2 to 5, more preferably from about 0.5 to 3 % w/w of the composition of the invention.

### Fatty Alcohols

Hair conditioning compositions of the invention will typically also incorporate a fatty alcohol. The combined use of fatty alcohols and cationic surfactants in conditioning compositions is believed to be especially advantageous, because this leads to the formation of a lamellar phase, in which the cationic surfactant is dispersed.

Representative fatty alcohols comprise from 8 to 22 carbon atoms, more preferably 16 to 22. Fatty alcohols are typically compounds containing straight chain alkyl groups. Examples of suitable fatty alcohols include cetyl alcohol, stearyl alcohol and mixtures thereof. The use of these materials is also advantageous in that they contribute to the overall conditioning properties of compositions of the invention.

The level of fatty alcohol in conditioners of the invention will generally range from 0.01 to 10, preferably from 0.1 to 8, more preferably from 0.2 to 7, most preferably from 0.3 to 6 % w/w by weight of the composition. The weight ratio of cationic surfactant to fatty alcohol is suitably from 1:1 to 1:10, preferably from 1:1.5 to 1:8, optimally from 1:2 to 1:5. If the weight ratio of cationic surfactant to fatty alcohol is too high, this can lead to eye irritancy from the composition. If it is too low, it can make the hair feel squeaky for some consumers.

The hair colour composition may comprise:
(a) A first component comprising the oxidative enzyme; and
(b) A second component comprising the hydrogen peroxide;
wherein the flavonoid of structure (I) and the compound of structure (II) is independently present in either the first or second components.

More generally, the hair colour composition may be in the form of a gel, cream, mask, ointment, mousse or lotion.

In a second aspect of the invention, a kit for colouring hair is provided, the kit for colouring hair comprising:
(a) A hair colour composition of the first aspect of the invention; and
(b) A pre- or post-treatment composition comprising a metal ion suitable for coordinating to the flavonoid of structure (I) or the product of the reaction of technical features (a), (b), (c) and (d) of the first aspect of the invention.

The metal ion of the kit for colouring hair may be selected from the group consisting of iron (II), iron (III), copper (I), copper (II), copper (III), aluminium (III), zinc (I), zinc (II), manganese (II), manganese (III), manganese (IV), manganese (V), manganese (VI) and manganese (VII).

In a third aspect of the invention, a method for colouring hair is provided, the method for colouring hair comprising the step of treating hair with the hair composition of the first aspect of the invention.

The method for colouring hair preferably comprises the steps of treating the hair successively with the first component and second component of the first aspect of the invention in any order.

The method for colouring hair preferably comprises the additional steps of:
(a) Treating the hair with the pre- or post-treatment composition of the second aspect of the invention; and
(b) Washing the hair;
wherein step (a) is completed prior to or after treatment of the hair with the hair colour composition of the first aspect of the invention; and
wherein step (b) is completed between step (a) and treatment of the hair with the hair colour composition of the first aspect of the invention.

### Example 1: Hair colour composition according to the invention.

### Materials

All materials were obtained from Sigma-Aldrich Company.

### Methods

### Treatment of hair fibres

Hair fibres were incubated at room temperature in a buffered solution (Britton-Robinson buffer, 40 mM, pH 6) containing a flavonoid (15 mM (+)-catechin or (-)-epicatechin; 5 mM of luteolin, taxifolin or quercetin), a catecholamine (epinephrine, isoprenaline or norepinephrine; 15, 50 or 100 mM) and horseradish peroxidase (Type VI, 274 units/mg solid; 0.1 mg/ml). Hydrogen peroxide was then added (0.3 % w/v final concentration) and the hair was incubated in this solution for 30 minutes. Hair fibres were then rinsed in water and washed with shampoo.

All controls included the same levels of hydrogen peroxide and horseradish peroxidase.

### Hair colour measurements

Natural white hair (Natural white 0.5G / 2" net round with epoxy, International Hair Importers & Products Inc, New York, US) colour was recorded with a spectrophotometer (Konica Minolta, CM-2600d) and the SpectraMagic NX software. Six L*a*b* measurements per sample were taken before and after the treatment.

### Results

Table 1 shows the dL*, da* and db* values for selected catecholamines of structure II consisting of DL-epinephrine hydrochloride, DL-isoprenaline hydrochloride and DL-norepinephrine hydrochloride controls in Britton-Robinson buffer (pH 6) at various concentrations.

**Table 1: dL*, da* and db* values for selected catecholamines of structure II consisting of DL-epinephrine hydrochloride, DL-isoprenaline hydrochloride and DL-norepinephrine hydrochloride controls in Britton-Robinson buffer (pH 6) at various concentrations (n = 6).**

| | | controls | | | | | |
|---|---|---|---|---|---|---|---|
| | | dL* | | da* | | db* | |
| | | Average | Std. error | Average | Std. error | Average | Std. error |
| Epinephrine hydrochloride, DL- | 15 mM | -1.36 | 0.48 | -0.31 | 0.13 | -0.76 | 0.36 |
| Epinephrine hydrochloride, DL- | 50 mM | -1.94 | 0.29 | -0.35 | 0.12 | -1.27 | 0.72 |
| Epinephrine hydrochloride, DL- | 100 mM | -2.13 | 0.39 | -0.26 | 0.08 | -1.19 | 0.42 |
| Isoprenaline hydrochloride. DL- | 15 mM | -1.37 | 0.80 | -0.75 | 0.16 | -0.07 | 0.32 |
| Isoprenaline hydrochloride. DL- | 50 mM | -4.17 | 0.94 | -0.37 | 0.16 | 1.29 | 0.90 |
| Isoprenaline hydrochloride. DL- | 100 mM | -6.72 | 0.66 | 0.48 | 0.17 | 4.02 | 0.60 |
| Norepinephrine hydrochloride, DL- | 15 mM | -2.19 | 0.38 | -0.39 | 0.08 | -2.32 | 0.40 |
| Norepinephrine hydrochloride, DL- | 50 mM | -7.46 | 0.41 | 0.15 | 0.11 | -3.59 | 0.34 |
| Norepinephrine hydrochloride, DL- | 100 mM | -9.62 | 1.46 | 0.18 | 0.24 | -4.21 | 0.46 |

Tables 2 to 6 show the dL*, da* and db* values for hair colour compositions comprising selected flavonoids of structure I consisting of respectively (+)-catechin or luteolin or quercetin or taxifolin or (-)-epicatechin each with the selected catecholamines of structure II set forth in Table 1. The concentrations of the selected flavonoids of structure I was 5 mM except for (+)-catechin and (-)-epicatechin which were at 15 mM. The selected catecholamines had concentrations of 15, 50 and 100 mM.

From the data in Table 2, it can be seen that the dL* for the combinations of (+)-catechin with the selected catecholamines was more negative than that of the respective controls.

**Table 2: dL*, da* and db* values for hair colour compositions comprising (+)-catechin and the selected catecholamines of structure II set forth in Table 1 (n = 6).**

| | | (+)-Catechin | | | | | |
|---|---|---|---|---|---|---|---|
| | | dL* | | da* | | db* | |
| | | Average | Std. error | Average | Std. error | Average | Std. error |
| Epinephrine hydrochloride, DL- | 15 mM | -11.88 | 0.72 | 4.38 | 0.32 | 2.86 | 0.70 |
| Epinephrine hydrochloride, DL- | 50 mM | -15.67 | 1.10 | 4.47 | 0.36 | 5.24 | 0.47 |
| Epinephrine hydrochloride, DL- | 100 mM | -15.14 | 0.77 | 4.33 | 0.29 | 7.54 | 0.82 |
| Isoprenaline hydrochloride. DL- | 15 mM | -9.95 | 0.97 | 2.78 | 0.27 | 2.88 | 0.35 |
| Isoprenaline hydrochloride. DL- | 50 mM | -17.04 | 1.36 | 4.95 | 0.63 | 4.87 | 0.53 |
| Isoprenaline hydrochloride. DL- | 100 mM | -16.87 | 0.46 | 4.76 | 0.23 | 5.97 | 1.00 |
| Norepinephrine hydrochloride, DL- | 15 mM | -11.19 | 0.77 | 1.75 | 0.20 | -2.22 | 0.62 |
| Norepinephrine hydrochloride, DL- | 50 mM | -14.24 | 1.09 | 1.52 | 0.19 | -4.76 | 0.49 |
| Norepinephrine hydrochloride, DL- | 100 mM | -18.32 | 2.19 | 1.17 | 0.33 | -5.74 | 0.54 |
| | | | | | | | |
| (+)-catechin control | 15 mM | -3.30 | 0.66 | 4.41 | 0.42 | 6.93 | 0.75 |

The data in Table 3 shows that the dL* for the combinations of luteolin with the selected catecholamines was more negative than that of the respective controls.

**Table 3: dL*, da* and db* values for hair colour compositions comprising luteolin and the selected catecholamines of structure II set forth in Table 1 (n = 6).**

| | | Luteolin | | | | | |
|---|---|---|---|---|---|---|---|
| | | dL* | | da* | | db* | |
| | | Average | Std. error | Average | Std. error | Average | Std. error |
| nephrine hydrochloride, DL- | 15 mM | -11.72 | 0.84 | 2.86 | 0.21 | 3.90 | 0.26 |
| nephrine hydrochloride, DL- | 50 mM | -15.22 | 0.54 | 3.84 | 0.37 | 8.03 | 0.74 |
| nephrine hydrochloride, DL- | 100 mM | -12.69 | 0.52 | 2.60 | 0.16 | 6.95 | 0.63 |
| Isoprenaline hydrochloride. DL- | 15 mM | -11.87 | 0.87 | 1.83 | 0.22 | 3.96 | 0.52 |
| Isoprenaline hydrochloride. DL- | 50 mM | -19.28 | 0.66 | 5.05 | 0.32 | 4.90 | 1.00 |
| Isoprenaline hydrochloride. DL- | 100 mM | -15.64 | 0.92 | 2.97 | 0.26 | 6.99 | 0.44 |
| Norepinephrine hydrochloride, DL- | 15 mM | -15.36 | 0.96 | 1.32 | 0.11 | -5.63 | 0.40 |
| Norepinephrine hydrochloride, DL- | 50 mM | -20.84 | 0.98 | 0.94 | 0.16 | -8.06 | 0.57 |
| Norepinephrine hydrochloride, DL- | 100 mM | -23.43 | 0.73 | 1.06 | 0.15 | -7.56 | 0.31 |
| | | | | | | | |
| Luteolin control | 5 mM | -1.67 | 0.72 | 0.62 | 0.18 | 0.12 | 0.56 |

The data in Table 4 also shows that the dL* for the combinations of quercetin with the selected catecholamines was more negative than that of the respective controls.

**Table 4: dL*, da* and db* values for hair colour compositions comprising quercetin and the selected catecholamines of structure II set forth in Table 1 (n = 6).**

| | | Quercetin | | | | | |
|---|---|---|---|---|---|---|---|
| | | dL* | | da* | | db* | |
| | | Average | Std. error | Average | Std. error | Average | Std. error |
| Epinephrine hydrochloride, DL- | 15 mM | -12.91 | 0.35 | 1.63 | 0.08 | 3.33 | 0.52 |
| Epinephrine hydrochloride, DL- | 50 mM | -13.36 | 0.45 | 1.48 | 0.22 | 2.56 | 0.28 |
| Epinephrine hydrochloride, DL- | 100 mM | -11.72 | 0.31 | 0.69 | 0.10 | 1.84 | 0.43 |
| Isoprenaline hydrochloride. DL- | 15 mM | -12.51 | 0.83 | 0.92 | 0.26 | 4.18 | 0.50 |
| Isoprenaline hydrochloride. DL- | 50 mM | -11.52 | 0.24 | 0.47 | 0.08 | 2.65 | 0.57 |
| Isoprenaline hydrochloride. DL- | 100 mM | -9.96 | 0.77 | -0.33 | 0.23 | 3.10 | 0.67 |
| Norepinephrine hydrochloride, DL- | 15 mM | -14.26 | 0.97 | -0.12 | 0.23 | -2.71 | 0.43 |
| Norepinephrine hydrochloride, DL- | 50 mM | -17.25 | 0.55 | -0.16 | 0.11 | -4.90 | 0.36 |
| Norepinephrine hydrochloride, DL- | 100 mM | -15.74 | 1.01 | -0.34 | 0.10 | -4.58 | 0.47 |
| | | | | | | | |
| Quercetin control | 5 mM | -5.89 | 0.27 | -0.20 | 0.10 | 1.33 | 0.44 |

The data in Table 5 shows that the dL* for the combinations of taxifolin with the selected catecholamines was more negative than that of the respective controls.

**Table 5: dL*, da* and db* values for hair colour compositions comprising taxifolin and the selected catecholamines of structure II set forth in Table 1 (n = 6).**

| | | Taxifolin | | | | | |
|---|---|---|---|---|---|---|---|
| | | dL* | | da* | | db* | |
| | | Average | Std. error | Average | Std. error | Average | Std. error |
| Epinephrine hydrochloride, DL- | 15 mM | -10.81 | 0.30 | 2.38 | 0.09 | 1.66 | 0.44 |
| Epinephrine hydrochloride, DL- | 50 mM | -12.09 | 0.85 | 2.92 | 0.18 | 2.24 | 0.70 |
| Epinephrine hydrochloride, DL- | 100 mM | -10.88 | 0.73 | 2.46 | 0.21 | 2.34 | 0.35 |
| Isoprenaline hydrochloride. DL- | 15 mM | -10.00 | 0.90 | 1.66 | 0.28 | 1.77 | 0.52 |
| Isoprenaline hydrochloride. DL- | 50 mM | -15.03 | 0.46 | 3.18 | 0.22 | 2.38 | 0.87 |
| Isoprenaline hydrochloride. DL- | 100 mM | -9.47 | 0.71 | 1.29 | 0.18 | 2.41 | 0.21 |
| Norepinephrine hydrochloride, DL- | 15 mM | -13.78 | 0.60 | 1.24 | 0.14 | -4.77 | 0.45 |
| Norepinephrine hydrochloride, DL- | 50 mM | -21.07 | 1.25 | 0.96 | 0.09 | -7.60 | 0.59 |
| Norepinephrine hydrochloride, DL- | 100 mM | -23.16 | 1.33 | 1.17 | 0.13 | -7.79 | 0.72 |
| | | | | | | | |
| Taxifolin control | 5 mM | -3.87 | 0.62 | 1.49 | 0.12 | -1.98 | 0.30 |

The data in Table 6 shows that the dL* for the combinations of (-)-epicatechin with the selected catecholamines was more negative than that of the respective controls.

**Table 6: dL*, da* and db* values for hair colour compositions comprising (-)-epicatechin and the selected catecholamines of structure II set forth in Table 1 (n = 6).**

| | | (-)-Epicatechin | | | | | |
|---|---|---|---|---|---|---|---|
| | | dL* | | da* | | db* | |
| | | Average | Std. error | Average | Std. error | Average | Std. error |
| Epinephrine hydrochloride, DL- | 15 mM | -10.71 | 0.72 | 4.05 | 0.25 | 3.83 | 0.45 |
| Epinephrine hydrochloride, DL- | 50 mM | -13.02 | 1.06 | 3.54 | 0.60 | 5.97 | 0.75 |
| Epinephrine hydrochloride, DL- | 100 mM | -12.81 | 1.29 | 3.22 | 0.61 | 6.28 | 0.47 |
| Isoprenaline hydrochloride, DL- | 15 mM | -8.85 | 0.46 | 2.45 | 0.45 | 3.90 | 0.72 |
| Isoprenaline hydrochloride, DL- | 50 mM | -13.72 | 0.84 | 3.35 | 0.32 | 4.78 | 0.33 |
| Isoprenaline hydrochloride, DL- | 100 mM | -11.94 | 0.80 | 2.47 | 0.46 | 4.52 | 0.76 |
| Norepinephrine hydrochloride, DL- | 15 mM | -8.10 | 1.14 | 1.98 | 0.27 | -0.16 | 0.33 |
| Norepinephrine hydrochloride, DL- | 50 mM | -9.16 | 0.60 | 1.26 | 0.09 | -1.79 | 0.38 |
| Norepinephrine hydrochloride, DL- | 100 mM | -14.63 | 1.12 | 1.61 | 0.19 | -3.43 | 0.26 |
| | | | | | | | |
| (-)-Epicatechin control | 15mM | -2.89 | 0.38 | 3.35 | 0.29 | 4.95 | 0.70 |

### Conclusions

Hair colour compositions comprising selected flavonoids of structure I consisting of respectively (+)-catechin or luteolin or quercetin or taxifolin or (-)-epicatechin each with the selected catecholamines of structure II consisting of DL-epinephrine hydrochloride, DL-isoprenaline hydrochloride and DL-norepinephrine hydrochloride at various concentrations in the presence of hydrogen peroxide and horseradish peroxidase darkened natural white hair to a greater extent than comparable compositions with either the aforementioned flavonoids or catecholamines.

### Example 2: Comparative example according to US 6 953 486 B2.

### Materials (not already mentioned in Example 1)

All materials were obtained from Sigma-Aldrich Company.

### Method

Hair colour composition substituting the catecholamine used in the invention with cysteine (as disclosed in US 6,953,486 B2), and combining L-DOPA (as the colouring agent precursor disclosed in US 6,953,486 B2) with cysteine (a combination specifically disclosed in US 6,953,486 B2) were prepared and compared with hair colour compositions of the invention. Relevant controls were prepared in the same manner as described in Example 1.

The colour uptake on hair was measured using the protocols described in Example 1.

### Results

Table 7 shows the dL*, da* and db* values for cysteine, DL-epinephrine hydrochloride, DL-isoprenaline hydrochloride and DL-norepinephrine hydrochloride controls in Britton-Robinson buffer (pH 6), with hydrogen peroxide and horseradish peroxidase, at various concentrations. Table 8 shows the dL*, da* and db* values for hair colour compositions comprising (+)-catechin-cysteine and (+)-catechin-catecholamines of structure II. The (+)-catechin control (15 mM) had a dL* value of -5.10 ± 0.23, and the cysteine control had dL* values of -0.34 ± 0.48, -0.37 ± 0.58 and 1.68 ± 0.25 for respectively 15, 50 and 100 mM. The dL* values for the combination of (+)-catechin and cysteine were -4.72 ± 0.37, -5.28 ± 0.69 and -2.45 ± 0.24 for cysteine concentrations of 15, 50 and 100 mM respectively. The (+)-catechin concentration was 15 mM in all cases.

Thus it is apparent that, in contrast to the hair colour compositions of the invention, the combination of (+)-catechin and cysteine does not produce a negative change in the L* value, i.e. a darkening effect.

**Table 7: dL*, da* and db* values for cysteine, DL-epinephrine hydrochloride, DL-isoprenaline hydrochloride and DL-norepinephrine hydrochloride controls in Britton-Robinson buffer (pH 6) at various concentrations (n = 6).**

| | | Controls | | | | | |
|---|---|---|---|---|---|---|---|
| | | dL | | da* | | db* | |
| | | Average | Std. error | Average | Std. error | Average | Std. error |
| Cysteine | 15 mM | -0.34 | 0.48 | -0.22 | 0.19 | -0.89 | 0.32 |
| Cysteine | 50 mM | -0.37 | 0.58 | -0.30 | 0.05 | -1.23 | 0.36 |
| Cysteine | 100 mM | 1.68 | 0.25 | -0.32 | 0.05 | -1.08 | 0.20 |
| Epinephrine hydrochloride, DL- | 15 mM | -0.43 | 0.50 | -0.05 | 0.26 | 1.30 | 0.37 |
| Epinephrine hydrochloride, DL- | 50 mM | -0.46 | 0.29 | -0.48 | 0.12 | 1.40 | 0.15 |
| Epinephrine hydrochloride, DL- | 100 mM | -1.85 | 0.28 | -0.32 | 0.07 | 0.96 | 0.29 |
| Isoprenaline hydrochloride, DL- | 15 mM | -1.12 | 0.64 | -0.72 | 0.23 | 0.05 | 0.42 |
| Isoprenaline hydrochloride, DL- | 50 mM | -2.33 | 0.71 | -0.94 | 0.18 | 0.92 | 0.55 |
| Isoprenaline hydrochloride, DL- | 100 mM | -1.39 | 0.46 | -1.28 | 0.11 | 2.08 | 0.53 |
| Norepinephrine hydrochloride, DL- | 15 mM | -0.57 | 0.69 | -0.08 | 0.11 | -1.39 | 0.31 |
| Norepinephrine hydrochloride, DL- | 50 mM | -3.92 | 1.39 | 0.12 | 0.10 | -2.21 | 0.38 |
| Norepinephrine hydrochloride, DL- | 100 mM | -4.95 | 0.31 | 0.18 | 0.09 | -2.54 | 0.47 |

**Table 8: dL*, da* and db* values for hair colour compositions comprising (+)-catechin-cysteine and (+)-catechin-catecholamines of structure II (n = 6).**

| | | (+)-Catechin | | | | | |
|---|---|---|---|---|---|---|---|
| | | dL | | da* | | db* | |
| | | Average | Std. error | Average | Std. error | Average | Std. error |
| Cysteine | 15 mM | -4.72 | 0.37 | 4.35 | 0.36 | 6.23 | 0.67 |
| Cysteine | 50 mM | -5.28 | 0.69 | 3.79 | 0.37 | 5.90 | 0.45 |
| Cysteine | 100 mM | -2.45 | 0.24 | 1.99 | 0.08 | 3.93 | 0.51 |
| Epinephrine hydrochloride, DL- | 15 mM | -12.46 | 0.44 | 5.20 | 0.33 | 5.72 | 0.68 |
| Epinephrine hydrochloride, DL- | 50 mM | -13.22 | 0.27 | 4.07 | 0.19 | 6.09 | 0.83 |
| Epinephrine hydrochloride, DL- | 100 mM | -12.88 | 0.60 | 3.44 | 0.33 | 10.14 | 0.62 |
| Isoprenaline hydrochloride, DL- | 15 mM | -10.82 | 0.45 | 3.41 | 0.15 | 3.34 | 0.14 |
| Isoprenaline hydrochloride, DL- | 50 mM | -11.86 | 0.51 | 3.00 | 0.07 | 6.57 | 0.54 |
| Isoprenaline hydrochloride, DL- | 100 mM | -14.30 | 1.40 | 3.53 | 0.22 | 7.96 | 0.25 |
| Norepinephrine hydrochloride, DL- | 15 mM | -11.50 | 0.66 | 2.46 | 0.16 | 0.75 | 0.52 |
| Norepinephrine hydrochloride, DL- | 50 mM | -13.41 | 0.43 | 1.65 | 0.11 | -3.98 | 1.76 |
| Norepinephrine hydrochloride, DL- | 100 mM | -17.59 | 0.37 | 1.43 | 0.13 | -5.14 | 0.62 |
| | | | | | | | |
| (+)-Catechin control | 15mM | -5.10 | 0.23 | 6.44 | 0.32 | 9.36 | 0.62 |

Table 9 shows dL*, da* and db* values for hair colour compositions comprising L-DOPA and cysteine. The L-DOPA control (at 15 mM) had a dL* of -1.51 ± 0.43. The dL* values for the combination of L-DOPA and cysteine were -1.46 ± 0.55, 0.42 ± 0.34 and 0.67 ± 0.30 for cysteine concentrations of 15, 50 and 100 mM respectively. The concentration of L-DOPA in all cases was 15 mM.

Thus it is apparent that, in contrast to the hair colour compositions of the invention, the combination of L-DOPA and cysteine does not produce a negative change in the L* value, i.e. a darkening effect.

**Table 9: dL*, da* and db* values for hair colour compositions comprising L-DOPA and cysteine (n = 6).**

| | | L-DOPA | | | | | |
|---|---|---|---|---|---|---|---|
| | | dL | | da* | | db* | |
| | | Average | Std. error | Average | Std. error | Average | Std. error |
| Cysteine | 15 mM | -1.46 | 0.55 | -0.36 | 0.07 | -2.34 | 0.18 |
| Cysteine | 50 mM | 0.42 | 0.34 | -0.34 | 0.12 | -0.84 | 0.48 |
| Cysteine | 100 mM | 0.67 | 0.30 | -0.50 | 0.12 | -0.91 | 0.23 |
| | | | | | | | |
| L-DOPA control | 15 mM | -1.51 | 0.43 | -0.36 | 0.08 | -2.43 | 0.36 |

### Conclusions

The hair colour compositions of the invention, when applied to natural white hair fibres, provided superior darkening than comparative compositions based on US 6,953,486 B2.

### Example 3: Molar ratio of flavonoid of structure (I) to compound of structure (II)

### Materials

All materials were obtained from Sigma-Aldrich Company.

### Method

The colour uptake on hair was measured using the protocols described in Example 1.

### Results

Table 10 shows the dL*, da* and db* values for selected catecholamines of structure II consisting of DL-epinephrine hydrochloride and DL-isoprenaline hydrochloride controls in Britton-Robinson buffer (pH 6) at various concentrations.

**Table 10: dL*, da* and db* values for selected catecholamines of structure II consisting of DL-epinephrine hydrochloride and DL-isoprenaline hydrochloride controls in Britton-Robinson buffer (pH 6) at various concentrations (n = 6).**

| | | Controls | | | | | |
|---|---|---|---|---|---|---|---|
| | | dL | | da* | | db* | |
| | | Average | Std. error | Average | Std. error | Average | Std. error |
| Epinephrine hydrochloride, DL- | 0.5 mM | 0.76 | 0.18 | -0.14 | 0.04 | -1.09 | 0.10 |
| Epinephrine hydrochloride, DL- | 1 mM | 0.36 | 0.22 | -0.23 | 0.06 | -0.19 | 0.23 |
| Epinephrine hydrochloride, DL- | 5 mM | -0.02 | 0.23 | -0.45 | 0.07 | -0.78 | 0.16 |
| Epinephrine hydrochloride, DL- | 15 mM | -1.02 | 0.26 | -0.45 | 0.06 | -0.82 | 0.20 |
| Epinephrine hydrochloride, DL- | 25 mM | -0.51 | 0.17 | -0.29 | 0.06 | 0.03 | 0.12 |
| Epinephrine hydrochloride, DL- | 50 mM | -1.04 | 0.23 | -0.35 | 0.07 | -0.27 | 0.28 |
| Epinephrine hydrochloride, DL- | 100 mM | -1.93 | 0.49 | -0.06 | 0.04 | -0.46 | 0.18 |
| Epinephrine hydrochloride, DL- | 200 mM | -2.03 | 0.18 | -0.14 | 0.03 | 0.80 | 0.37 |
| | | | | | | | |
| Isoprenaline hydrochloride, DL- | 0.5 mM | 1.29 | 0.12 | -0.27 | 0.03 | -0.57 | 0.25 |
| Isoprenaline hydrochloride, DL- | 1 mM | 1.22 | 0.17 | -0.35 | 0.08 | -0.04 | 0.24 |
| Isoprenaline hydrochloride, DL- | 5 mM | 0.24 | 0.16 | -0.61 | 0.05 | -0.59 | 0.26 |
| Isoprenaline hydrochloride, DL- | 15 mM | 0.07 | 0.28 | -0.62 | 0.07 | -0.36 | 0.28 |
| Isoprenaline hydrochloride, DL- | 25 mM | -0.57 | 0.22 | -0.57 | 0.05 | 0.09 | 0.20 |
| Isoprenaline hydrochloride, DL- | 50 mM | -1.49 | 0.28 | -0.52 | 0.09 | 0.41 | 0.39 |
| Isoprenaline hydrochloride, DL- | 100 mM | -2.35 | 0.39 | -0.58 | 0.04 | 0.70 | 0.25 |
| Isoprenaline hydrochloride, DL- | 200 mM | -5.09 | 0.33 | -0.45 | 0.16 | 0.01 | 0.37 |

Tables 11 and 12 show the dL*, da* and db* values for hair colour compositions comprising selected flavonoids of structure I consisting of respectively (+)-catechin or luteolin, each with the selected catecholamines of structure II set forth in Table 10. The concentrations of the selected flavonoids of structure I was 5 mM. The selected catecholamines had concentrations of 0.5, 1, 5, 15, 25, 50, 100 and 200 mM.

From the data in Table 11, it can be seen that the dL* for the combinations of (+)-catechin with the selected catecholamines was more negative when the molar ratio of flavonoid to catecholamine was between 1:1 and 1:20, and even more negative when the ratio was between 1:3 and 1:10.

**Table 11: dL*, da* and db* values for hair colour compositions comprising (+)-catechin and the selected catecholamines of structure II set forth in Table 10 (n = 6).**

| | | (+)-Catechin | | | | | |
|---|---|---|---|---|---|---|---|
| | | dL | | da* | | db* | |
| | | Average | Std. error | Average | Std. error | Average | Std. error |
| Epinephrine hydrochloride, DL- | 0.5 mM | -6.11 | 0.52 | 7.08 | 0.32 | 6.93 | 0.51 |
| Epinephrine hydrochloride, DL- | 1 mM | -6.54 | 0.55 | 6.79 | 0.31 | 6.79 | 0.29 |
| Epinephrine hydrochloride, DL- | 5 mM | -9.41 | 0.50 | 5.75 | 0.39 | 5.10 | 0.31 |
| Epinephrine hydrochloride, DL- | 15 mM | -14.25 | 0.60 | 5.28 | 0.24 | 3.99 | 0.29 |
| Epinephrine hydrochloride, DL- | 25 mM | -13.12 | 0.76 | 5.07 | 0.25 | 4.66 | 0.47 |
| Epinephrine hydrochloride, DL- | 50 mM | -14.89 | 0.41 | 4.54 | 0.13 | 6.63 | 0.88 |
| Epinephrine hydrochloride, DL- | 100 mM | -13.68 | 0.34 | 3.39 | 0.23 | 9.19 | 0.67 |
| Epinephrine hydrochloride, DL- | 200 mM | -11.80 | 0.37 | 3.20 | 0.19 | 9.06 | 0.55 |
| | | | | | | | |
| Isoprenaline hydrochloride, DL- | 0.5 mM | -7.17 | 0.54 | 6.27 | 0.30 | 6.74 | 0.48 |
| Isoprenaline hydrochloride, DL- | 1 mM | -5.88 | 0.57 | 4.67 | 0.22 | 4.78 | 0.23 |
| Isoprenaline hydrochloride, DL- | 5 mM | -8.25 | 0.79 | 3.71 | 0.31 | 3.43 | 0.35 |
| Isoprenaline hydrochloride, DL- | 15 mM | -12.16 | 0.80 | 3.30 | 0.29 | 2.42 | 0.43 |
| Isoprenaline hydrochloride, DL- | 25 mM | -13.44 | 0.64 | 4.13 | 0.20 | 3.19 | 0.30 |
| Isoprenaline hydrochloride, DL- | 50 mM | -15.26 | 0.45 | 4.61 | 0.29 | 5.60 | 0.44 |
| Isoprenaline hydrochloride, DL- | 100 mM | -15.61 | 0.42 | 4.04 | 0.31 | 6.58 | 0.64 |
| Isoprenaline hydrochloride, DL- | 200 mM | -11.39 | 0.53 | 2.28 | 0.15 | 6.11 | 0.53 |
| | | | | | | | |
| (+)-Catechin control | 5 mM | -3.65 | 0.26 | 5.78 | 0.13 | 5.49 | 0.45 |

From the data in Table 12, it can be seen that the dL* for the combinations of luteolin with the selected catecholamines was more negative when the molar ratio of flavonoid to catecholamine was between 1:1 and 1:20, and even more negative when the ratio was between 1:3 and 1:10.

**Table 12: dL*, da* and db* values for hair colour compositions comprising luteolin and the selected catecholamines of structure II set forth in Table 10 (n = 6).**

| | | Luteolin | | | | | |
|---|---|---|---|---|---|---|---|
| | | dL | | da* | | db* | |
| | | Average | Std. error | Average | Std. error | Average | Std. error |
| Epinephrine hydrochloride, DL- | 0.5 mM | -2.80 | 0.24 | 0.27 | 0.07 | 0.54 | 0.23 |
| Epinephrine hydrochloride, DL- | 1 mM | -3.66 | 0.57 | 0.86 | 0.10 | 1.73 | 0.16 |
| Epinephrine hydrochloride, DL- | 5 mM | -5.56 | 0.29 | 0.89 | 0.06 | 2.48 | 0.19 |
| Epinephrine hydrochloride, DL- | 15 mM | -9.51 | 0.59 | 2.07 | 0.26 | 3.77 | 0.07 |
| Epinephrine hydrochloride, DL- | 25 mM | -11.39 | 0.36 | 2.42 | 0.22 | 5.07 | 0.41 |
| Epinephrine hydrochloride, DL- | 50 mM | -11.60 | 0.60 | 2.64 | 0.17 | 5.26 | 0.37 |
| Epinephrine hydrochloride, DL- | 100 mM | -9.95 | 0.31 | 2.15 | 0.15 | 7.63 | 0.29 |
| Epinephrine hydrochloride, DL- | 200 mM | -8.24 | 0.15 | 1.48 | 0.14 | 4.21 | 0.24 |
| | | | | | | | |
| Isoprenaline hydrochloride, DL- | 0.5 mM | -2.27 | 0.50 | 0.67 | 0.11 | 0.92 | 0.34 |
| Isoprenaline hydrochloride, DL- | 1 mM | -2.98 | 0.44 | 0.73 | 0.09 | 1.24 | 0.21 |
| Isoprenaline hydrochloride, DL- | 5 mM | -6.05 | 0.59 | 0.30 | 0.07 | 3.29 | 0.31 |
| Isoprenaline hydrochloride, DL- | 15 mM | -8.45 | 0.64 | 0.99 | 0.21 | 4.07 | 0.42 |
| Isoprenaline hydrochloride, DL- | 25 mM | -11.72 | 0.35 | 2.50 | 0.09 | 6.80 | 0.46 |
| Isoprenaline hydrochloride, DL- | 50 mM | -13.31 | 0.16 | 2.44 | 0.10 | 6.59 | 0.70 |
| Isoprenaline hydrochloride, DL- | 100 mM | -11.07 | 0.37 | 1.76 | 0.13 | 6.53 | 0.61 |
| Isoprenaline hydrochloride, DL- | 200 mM | -9.24 | 0.38 | 0.88 | 0.13 | 7.66 | 0.54 |
| | | | | | | | |
| Luteolin control | 5 mM | -2.16 | 0.65 | 0.65 | 0.15 | 0.06 | 0.28 |

### Conclusions

The hair colour compositions of the invention, when applied to natural white hair fibres, provided darker colours (i.e. a lower dL* value) when the molar ratio of a flavonoid of structure I to a compound of structure II was between 1:1 and 1:20, and even darker colours when the ratio was between 1:3 and 1:10.

## Claims

1. A hair colour composition comprising:
(a) A flavonoid of structure (I): 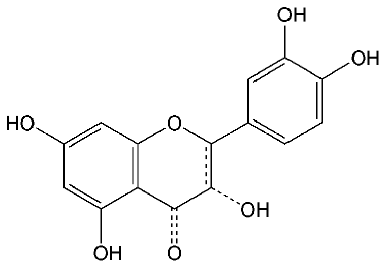
(b) A catecholamine of structure (II): 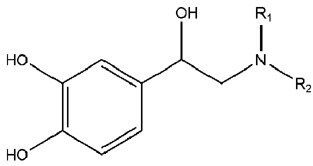 wherein R₁ and R₂ are independently H, C₁₋₅, C₁₋₃, C₁₋₂ branched or unbranched, substituted or unsubstituted alkyl moity, wherein the substituent is a halide, -OH, -NH₂ or =O;
(c) A peroxidase enzyme; and
(d) A hydrogen peroxide generator or hydrogen peroxide.

2. A hair colour composition according to claim 1 wherein the flavonoid is selected from the group consisting of catechin, luteolin, quercetin, taxifolin and epicatechin.

3. A hair colour composition according to claim 1 or claim 2 wherein the flavonoid is (+)-catechin or (-)-catechin.

4. A hair colour composition according to claim 1 or claim 2 wherein the flavonoid is (+)-epicatechin or (-)-epicatechin

5. A hair colour composition according to any one of the preceding claims wherein R₁ is -H.

6. A hair colour composition according to any one of the preceding claims wherein R₂ is a C₁₋₃ substituted or unsubstituted alkyl group or -H.

7. A hair colour composition according to claim 6 wherein R₂ is a methyl group or -H.

8. A hair colour composition according to any one of claims 1 to 4 wherein the catecholamine is selected from the group consisting of epinephrine, isoprenaline and norepinephrine.

9. A hair colour composition according to any one of the preceding claims, wherein the peroxidase is a non-animal haem peroxidase from class II (fungi) or class III (plants and algae).

10. A hair colour composition according to claim 9, wherein the peroxidase is obtained from the group consisting of Arabidopsis thaliana, horse radish, barley, peanut soy bean, tobacco, and turnip (plants), Chlorophyta spirogyra (green algae), Arthromyces ramosus and Corprinus cinereus (fungi).

11. A hair colour composition according to claim 10 wherein the peroxidase is horse radish peroxidase or soy bean peroxidase.

12. A hair colour composition according to any one of the preceding claims comprising 0.01 to 10, preferably 0.1 to 5 % w/w flavonoid of structure (I).

13. A hair colour composition according to any one of the preceding claims comprising 0.01 to 10, preferably 0.1 to 5 % w/w compound of structure (II).

14. A hair colour composition according to any one of the preceding claims comprising 0.0001 to 3, preferably 0.001 to 1, most preferably 0.01 to 1 % w/w hydrogen peroxide.

15. A hair colour composition according to any one of the preceding claims comprising 0.0001 to 5, preferably 0.001 to 1 % w/w peroxidase.

16. A hair colour composition according to any one of the preceding claims, wherein the hydrogen peroxide generator comprises a hydrogen peroxide generating oxidase, a substrate and oxygen.

17. A hair colour composition according to claim 16, wherein the hydrogen peroxide generating oxidase is selected from the group consisting of (S)-2-hydroxy acid oxidase, D-galactose oxidase, glucose oxidase, coniferyl alcohol oxidase, glycolate oxidase, hexose oxidase, oxalate oxidase, amino acid oxidase and L-galactonolactone oxidase and the respective substrate is selected from the group consisting of (S)-2-hydroxy acid, D-galactose, glucose, coniferyl alcohol, α-hydroxy acids, D-glucose, oxalic acid, amino acid and L-galactono-1,4-lactone.

18. A hair colour composition according to any one of the preceding claims, wherein the hydrogen peroxide generator is selected from the group consisting of (S)-2-hydroxy acid with (S)-2-hydroxy acid oxidase, D-galactose with D-galactose oxidase, glucose with glucose oxidase, coniferyl alcohol with coniferyl alcohol oxidase, α-hydroxy acids with glycolate oxidase, D-glucose with hexose oxidase, oxalic acid with oxalate oxidase, and L-galactono-1,4-lactone with L-galactonolactone oxidase, amino acid oxidase with amino acids, all in the presence of oxygen.

19. A hair colour composition according to any one of the preceding claims wherein the molar ratio of flavonoid of structure (I) to compound of structure (II) is 1:1 to 1:20, preferably 1:2-1:15, most preferably 1:3 to 1:10.

20. A hair colour composition according to any one of the preceding claims wherein the pH is 5-9, preferably 5-8, most preferably 5-7.

21. A hair colour composition according to any one of the preceding claims comprising:
(a) A first component comprising the oxidative enzyme; and
(b) A second component comprising the hydrogen peroxide;
wherein the flavonoid of structure (I) and the compound of structure (II) is independently present in either the first or second components.

22. A kit for colouring hair comprising:
(a) A hair colour composition according to any one of the preceding claims; and
(b) A pre- or post-treatment composition comprising a metal ion suitable for coordinating to the flavonoid of structure (I) or the product of the reaction of technical features (a), (b), (c) and (d) of claim 1.

23. A kit according to claim 22, wherein the metal ion is selected from the group consisting of iron (II), iron (III), copper (I), copper (II), copper (III), aluminium (III), zinc (I), zinc (II), manganese (II), manganese (III), manganese (IV), manganese (V), manganese (VI) and manganese (VII).

24. A method for colouring hair comprising the step of treating hair with the hair composition of any one of claims 1 to 21.

25. A method for colouring hair according to claim 24, the method comprising the steps of treating the hair successively with the first component and second component of claim 21 in any order.

26. A method for colouring hair according to claims 24 or 25 comprising the additional steps of:
(a) Treating the hair with the pre- or post-treatment composition of any one of claims 22 or 23; and
(b) Washing the hair;
wherein step (a) is completed prior to or after treatment of the hair with the hair colour composition; and
wherein step (b) is completed between step (a) and treatment of the hair with the hair colour composition.
